# FASCICULE DE BREVET EUROPEEN

(11) **EP 3 370 821 B1**
(45) Date de publication et mention de la délivrance du brevet: **07.08.2019**
(21) Numéro de dépôt: 16809132.0
(22) Date de dépôt: 04.11.2016
(51) Int. Cl.: A61N 1/30, A61N 1/32, A61N 1/04

(54) **DISPOSITIF D'APPLICATION D'UN PRODUIT A DISTRIBUER SUR LA PEAU D'UN UTILISATEUR PAR IONTOPHORESE**
VORRICHTUNG ZUM AUFTRAGEN EINES PRODUKTES ZUR VERTEILUNG AUF DER HAUT EINES BENUTZERS DURCH IONTOPHORESE
DEVICE FOR APPLYING A PRODUCT TO BE DISTRIBUTED ON THE SKIN OF A USER BY IONTOPHORESIS

(30) Priorité: 06.11.2015 FR 1560679
(43) Date de publication de la demande: 12.09.2018
(73) Titulaire: SEB S.A., 69130 Ecully (FR)
(72) Inventeur: MANDICA, Franck, 69340 Francheville (FR); SABATTIER, Johan, 69440 Mornant (FR)
(74) Mandataire: Bourrières, Patrice
(86) Numéro de dépôt international: PCT/FR2016/052868
(87) Numéro de publication internationale: WO 2017/077255

(56) Documents cités:
- EP-A2- 0 942 278
- WO-A2-02/085451
- FR-A1- 2 980 370
- US-A1- 2002 161 323

## Description

### 1. Domaine de l'invention

L'invention concerne le domaine des dispositifs permettant l'application d'un produit cosmétique ou thérapeutique sur la peau d'un utilisateur. En particulier, elle vise les dispositifs utilisant le principe de la iontophorèse pour améliorer la délivrance d'un principe actif d'un produit à travers les différentes couches de la peau.

### 2. Art antérieur

Il est connu un dispositif d'application d'un produit à distribuer sur la peau par iontophorèse destiné à améliorer la délivrance d'un principe actif d'un produit à travers la peau. Un tel dispositif est connu du document FR 2 619 308 lequel comprend un corps et une tête d'application fixée sur ledit corps. Le corps loge un réservoir pour le produit à appliquer, un générateur de courant électrique ainsi qu'une première et une deuxième électrodes recevant un courant électrique du générateur de courant. La première électrode recouvre une paroi du corps tandis que la seconde électrode est en contact permanent avec le produit contenu dans le réservoir. La tête d'application comporte une semelle équipée de logements dans lesquels chacun reçoit une bille et coopérant avec le réservoir de produit.

L'un des problèmes liés à ce dispositif d'application de produit à distribuer réside dans le fait que la première électrode est située sur la surface du corps du dispositif et que la deuxième électrode est localisée dans le réservoir qui est à proximité de la tête d'application ce qui crée des courants de fuite entre la première et la deuxième électrodes. Ces courants de fuite passent principalement par le produit du fait de la présence de la deuxième électrode dans le réservoir ce qui implique qu'un très faible courant traverse la peau pour permettre la pénétration du produit et ses principes actifs à travers celle-ci.

### 3. Objectifs de l'invention

L'invention a notamment pour objectif de pallier tout ou partie des inconvénients de l'art antérieur.

Un objectif de l'invention est de fournir un dispositif d'application d'un produit à distribuer par iontophorèrese sur la peau d'un utilisateur permettant les courant de fuite dans le réservoir contenant le produit tout en étant simple de fabrication et compact.

### 4. Résumé de l'invention

Ces objectifs sont atteints grâce à un dispositif d'application d'un produit à distribuer sur une peau d'un utilisateur par iontophorèse comprenant:
- un corps destiné à recevoir le produit à distribuer comprenant un générateur de courant électrique; et,
- une tête d'application montée sur le corps , la tête d'application comportant :
   o au moins un moyen de distribution du produit à distribuer sur la peau ; et
   o au moins une première et une deuxième électrodes séparées par une zone inter-électrode, les premières et les deuxièmes électrodes étant en retrait de ladite zone inter-électrodes et recevant un courant électrique dudit générateur.

Cette solution permet de résoudre les problèmes précités. En particulier, une telle configuration du dispositif d'application permet d'avoir un appareil compact, simple, et facile à manipuler et utiliser. Par ailleurs, le fait d'avoir les première et deuxième électrodes placées dans la tête d'application permet, d'une part de réduire la distance entre les première et deuxième électrodes ce qui entraine l'application d'un courant de plus faible intensité, et d'autre part, de maitriser le trajet du courant et la pénétration des principes actifs à travers la peau de l'utilisateur. De la sorte, il est possible de cibler la zone de la peau à traiter et limiter les courants de fuite.

De manière avantageuse, la tête d'application est amovible par rapport au corps de sorte à favoriser le nettoyage ou le remplacement de pièces défectueuses.

Selon une caractéristique de l'invention, la tête d'application comprend au moins une cavité de distribution.

Selon une caractéristique avantageuse, mais de manière facultative, les première et deuxième électrodes sont situées dans un même plan.

De manière plus précise, la zone inter-électrode est située dans un plan parallèle audit plan des première et deuxième électrodes, le plan des première et deuxième électrodes et le plan de la zone inter-électrode étant situés à une distance prédéterminée l'un par rapport à l'autre. Ainsi, la disposition des première et deuxième électrodes en retrait de la zone inter-électrode permet d'éviter un contact direct de celles-ci avec la peau de l'utilisateur ce qui empêche les irritations ou picotements électriques. Cela permet de réduire la quantité de produit pouvant séjourner/s'accumuler entre le plan des première et deuxième électrodes et le plan de la zone inter-électrode. En outre cette configuration permet de favoriser le passage de courant dans la peau et non pas à travers la formule située sur le plan des première et deuxième électrodes pour iontophorèse.

De manière avantageuse, mais facultativement, la distance prédéterminée entre le plan des première et deuxième électrodes et le plan de la zone inter-électrode est comprise entre 0.3 et 1.3 mm.

Selon une configuration avantageuse, mais non limitative, la zone inter-électrode comprend une surface d'application destinée à être en contact avec la peau de l'utilisateur. Cette configuration empêche également la formation d'un film épais de produit entre les première et deuxième électrode où le courant électrique circulerait alors préférentiellement.

Selon encore une caractéristique de l'invention, la zone inter-électrode présente une distance minimale prédéterminée entre la première électrode et la deuxième électrode. Cette distance minimale prédéterminée peut être comprise entre 5 et 20 mm. Cet agencement permet, d'une part de déterminer la zone de traitement et en particulier la profondeur de pénétration du courant et des principes actifs dans la peau de l'utilisateur.

Selon encore une caractéristique de l'invention, la tête d'application comprend un circuit de distribution comprenant des canaux de distribution distribuant le produit vers le moyen de distribution du produit.

Selon encore une caractéristique de l'invention, la première électrode et la deuxième électrode sont agencées en aval du circuit de distribution. Ainsi, le produit est distribué au droit des première et deuxième électrodes de manière à optimiser la quantité de principe actif du produit conduite dans la zone de traitement de la peau de l'utilisateur.

Selon une caractéristique de l'invention, la première électrode et la deuxième électrode comprennent chacune une paroi munie de perforations de sorte à permettre la circulation du produit vers la peau de l'utilisateur.

Selon un mode de réalisation, le moyen de distribution comprend un ou des orifices de sortie, les perforations des première et deuxième électrodes peuvent constituer le ou les orifices de sortie du produit vers la peau de l'utilisateur.

Selon un autre mode de réalisation, une plaque munie de perforations peut être agencée dans la tête d'application, le moyen de distribution comprenant un ou des orifices de sortie et les perforations constituant le ou les orifices de sortie du produit vers la peau de l'utilisateur.

Selon un mode de réalisation de l'invention, le produit peut être contenu dans une cartouche connectée de manière amovible au corps du dispositif. La disposition du produit à distribuer dans une cartouche permet un réapprovisionnement de manière facile et simple. Aussi, différents types de produits avec des applications cosmétiques et thérapeutiques peuvent être utilisés avec le dispositif d'application sans avoir à nettoyer le corps à chaque utilisation. Par ailleurs, la cartouche permet de maitriser la distribution du produit sur la peau de l'utilisateur.

Selon une caractéristique de l'invention, le corps peut comprendre au moins un moyen d'extraction du produit du corps vers la tête d'application. En particulier, ce moyen d'extraction peut être à commande manuelle ou motorisée.

Selon une caractéristique de l'invention, la tête d'application comprend un moyen d'isolation électrique lequel est interposé fluidiquement entre la première électrode et la deuxième électrode, ledit moyen d'isolation électrique étant configuré de manière à autoriser ou interdire le passage du courant électrique depuis les première et deuxième électrodes vers le corps. Cet agencement permet également la limitation des courants de fuite entre le corps et les première et deuxième électrodes. Ainsi, le dispositif d'application est alors plus sûr et plus efficace pour l'utilisateur.

Selon une caractéristique de l'invention, le moyen d'isolation électrique est propre à occuper :
- une position fermée dans laquelle le courant circule uniquement entre les première et deuxième électrodes; et,
- une position ouverte dans laquelle le courant circule entre les première et deuxième électrodes et dans les canaux de distribution de la tête d'application.

De la sorte, cette configuration permet de contrôler la circulation du produit et notamment celle du courant dans le corps du dispositif et notamment dans le produit qui est conducteur pour éviter les courants de fuite.

Selon encore une caractéristique de l'invention, le moyen d'isolation électrique prend la position ouverte lorsqu'une pression à l'intérieur du moyen d'isolation électrique atteint un seuil prédéterminé. De la sorte lorsqu'il n'est pas nécessaire d'approvisionner en produit (produit disponible dans la cavité de distribution de la tête), il n'y a pas de circulation de courant. De plus, cela permet de mettre sous pression le produit à distribuer lorsqu'il doit alimenter les cavités, ce qui l'aide à remplir les cavités de distribution.

Selon encore une caractéristique de l'invention, le moyen d'isolation électrique est disposé sur un des canaux de distribution menant vers la première électrode. Une telle configuration permet d'éviter que les lignes de courant ne passent de la première électrode à la deuxième électrode à l'intérieur de la tête d'application et d'interdire le passage du courant vers le corps.

Selon encore un autre mode de réalisation, la première électrode est disposée au centre de la tête et la deuxième électrode est disposée vers la périphérie de la tête afin de faciliter le montage et l'assemblage de la tête d'application.

De manière avantageuse mais non limitativement, le moyen d'isolation est un clapet. Le clapet permet d'autoriser ou d'arrêter de manière simple et efficace la circulation d'un fluide tout en étant étanche.

Selon encore un autre mode de réalisation, le dispositif d'application peut comprendre une sonde de température installée dans la tête. Cette sonde permet de surveiller la température de la peau de l'utilisateur afin d'éviter des lésions pouvant survenir lorsque l'intensité du courant est trop élevée.

### 5. Liste des figures

D'autres caractéristiques et avantages innovants ressortiront de la description ci-après, fournie à titre indicatif et nullement limitatif, en référence aux dessins annexés, dans lesquels :
- La figure 1 est une vue en perspective d'un dispositif d'application de produit à distribuer sur la peau d'un utilisateur par iontophorèse selon l'invention;
- La figure 2 est une vue en en coupe transversale longitudinale du dispositif d'application de produit selon le plan de coupe A-A de la figure 1.
- La figure 3 est une vue de dessus et en perspective d'un exemple de tête d'application d'un dispositif d'application de produit selon l'invention ;
- La figure 4 est une vue de dessous de l'exemple de tête d'application illustrée sur la figure 3 ;
- La figure 5 est une vue en perspective et éclatée d'un exemple de tête d'application d'un dispositif d'application selon l'invention.
- Les figures 6 et 7 illustrent de manière schématique et plus en détail une vue en coupe de deux exemples de tête d'application selon l'invention ;
- Les figures 8 à 10 représentent de manière schématique des modes de réalisation de l'agencement de première et deuxième électrodes dans la tête d'application ; et
- Les figures 11 à 13 représentent un exemple de moyen d'isolation électrique à interposer entre la première électrode et la deuxième électrode;
- La figure 14 est vue en coupe partielle et de détail d'un exemple de tête d'application montée et connectée sur un corps de dispositif d'application lequel comprend une cartouche selon l'invention ;
- La figure 15 est un diagramme bloc représentant les communications électriques entre un circuit électronique de commande, un générateur de courant et les première et deuxième électrodes ; et
- La figure 16 illustre une vue de dessus et en perspective d'une portion de connexion du corps du dispositif destinée à se connecter avec une tête d'application selon l'invention.

### 6. Description détaillée

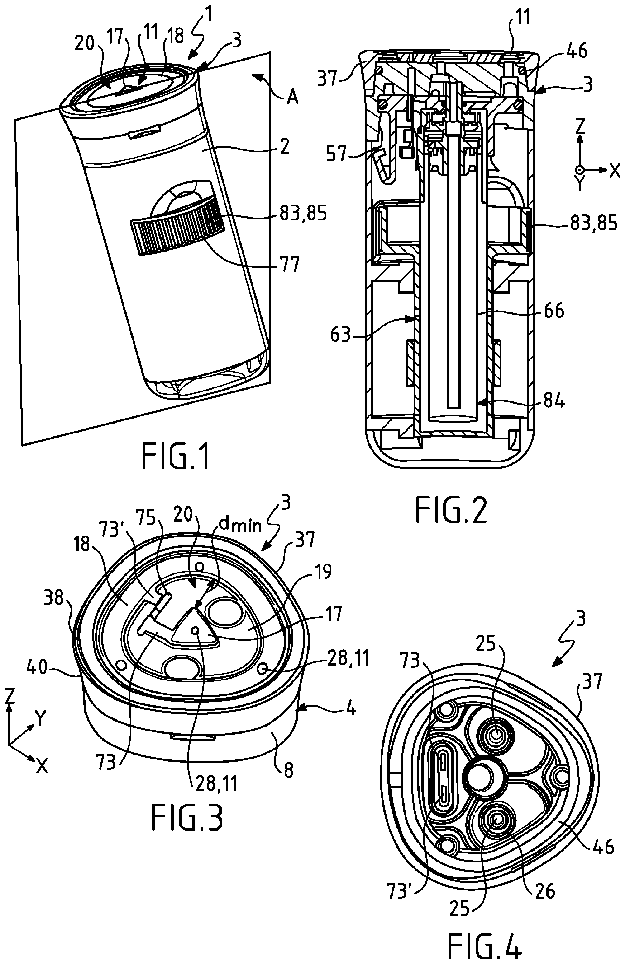

En référence à la figure 1, le dispositif 1 d'application d'un produit à distribuer sur la peau d'un utilisateur par iontophorèse selon l'invention est destiné au soin cosmétique et/ou thérapeutique de la peau de l'utilisateur. Le dispositif 1 ici utilise le principe d'iontophorèse, un principe du type électrophorèse pour favoriser et faciliter la pénétration du produit et de ses principes actifs à travers la peau de l'utilisateur. L'iontophorèse ici est l'application d'un champ électrique à travers la peau agissant comme une force motrice pour permettre le déplacement des ions du produit.
La peau est traitée de manière non invasive.

Le dispositif comprend un corps 2 formant un organe de préhension du dispositif 1 et une tête d'application 3 montée sur le corps 2. Tel que visible à la figure 2, le dispositif comprend au moins un moyen de stockage 84 du produit à distribuer sur la peau. De manière avantageuse, mais non limitativement, le corps 2 comprend le moyen de stockage 84 du produit à distribuer. Quant à la tête d'application 3 celle-ci comprend au moins un moyen de distribution 11 du produit à distribuer sur la peau de l'utilisateur.

On entend par le terme « produit » au sens de l'invention, un produit se présentant sous forme d'un fluide tel qu'un liquide et/ou une composition aqueuse.

En référence aux figures 2 à 7, la tête d'application 3 est montée sur le corps 2 de manière amovible de sorte à permettre un remplacement de celle-ci en cas de détérioration et/ou d'endommagement, et aussi pour faciliter son nettoyage. La tête d'application 3 telle qu'illustrée sur les figures 3 et 4 comprend une interface supérieure destinée à être orientée vers la peau de l'utilisateur et une interface inférieure opposée destinée à être connectée avec le corps 2 du dispositif d'application de produit.

Pour faciliter la compréhension de l'invention, nous considérons que la tête d'application 3 s'étend suivant un axe longitudinal Z ici vertical. Est également représenté un axe horizontal X qui est perpendiculaire à l'axe longitudinal vertical Z et à un axe transversal Y de sorte que ces trois axes X, Y, Z forment un repère orthogonal direct tel qu'illustré sur les figures 2 et 3 par exemple. Les termes « inférieur », « supérieur », « haut », « bas », « dessus », et «dessous» sont définis par rapport à l'axe longitudinal vertical Z et le terme « latéral » est défini par rapport à l'axe horizontal X.

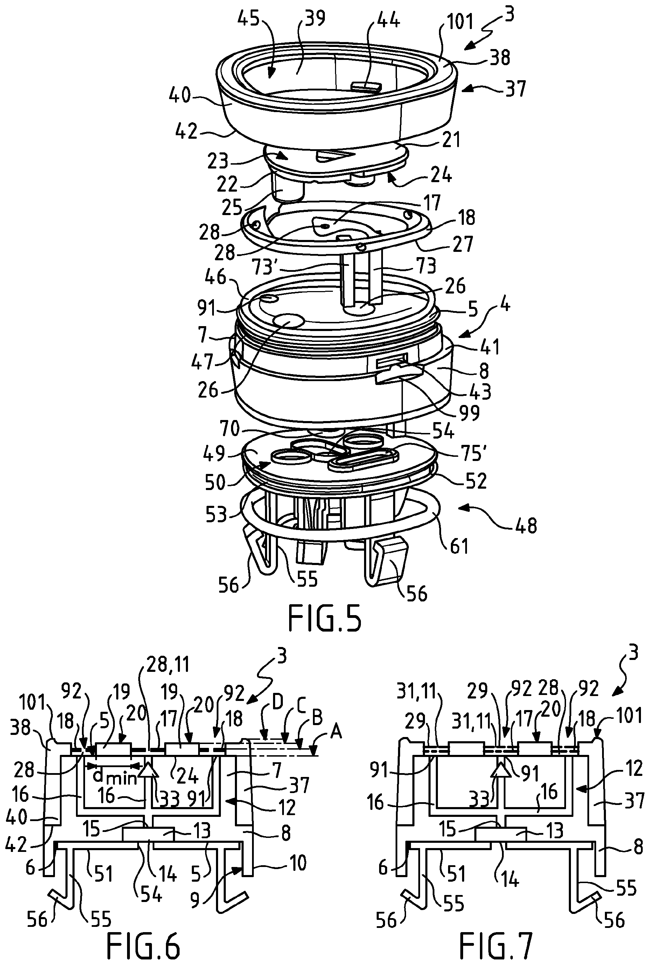

Plus précisément illustré sur les figures 5, 6 et 7, la tête d'application 3 comprend une embase 4 comportant une surface supérieure 5 et une surface inférieure 6 reliées par une paroi 7. Cette paroi 7 est prolongée par une jupe latérale 8 qui s'étend vers le bas, selon l'axe Z, à partir de la périphérie de la paroi 7. La jupe latérale 8 présente une surface interne 9 et une surface externe 10 opposée suivant sa longueur. L'embase 4 présente une section générale sensiblement triangulaire. Bien entendu, l'embase 4 peut présenter une section circulaire ou autre dès lors que celle-ci soit compatible avec le corps du dispositif 1 d'application.

Sur les figures 6 et 7, l'embase 4 comporte également un circuit de distribution 12 du produit à distribuer agencé entre la surface supérieure 5 et la surface inférieure 6. En particulier, le circuit de distribution 12 comporte, à l'endroit de la surface inférieure 6 de l'embase 4, un réservoir tampon 13 destiné à recevoir, voire collecter du produit en provenance du corps 2 du dispositif 1 d'application. Le réservoir tampon 13 présente une entrée 14 recevant le produit à distribuer du corps 2 et une sortie 15 permettant la circulation du produit vers le moyen de distribution 11 du produit sur la peau. Pour permettre la circulation du produit vers le moyen de distribution 11, le circuit de distribution 12 comporte des canaux de distribution 16 reliant le moyen de distribution 11 du produit et le réservoir tampon 13. Les canaux de distribution 16 sont formés dans la paroi 7 de l'embase 4. Ceux-ci débouchent sur la surface supérieure 5 de l'embase 4 par des orifices de distributions 91 et permettent en particulier de relier la sortie 15 du réservoir tampon 13 avec ces orifices de distribution 91. De manière avantageuse, mais non limitativement, chaque orifice de distribution 91 communique fluidiquement avec un canal de distribution 16 du circuit de distribution 12.

Le dispositif d'application comprend au moins une première électrode 17 et au moins une deuxième électrode 18 pour iontophorèse. Ces première et deuxième électrodes 17, 18 sont installées chacune dans au moins une cavité de distribution 92 que comprend le dispositif d'application 1 et propre à recevoir du produit à distribuer. En particulier, la cavité de distribution 92 est formée dans la tête d'application 3 et est en communication fluidique avec au moins un orifice de distribution 91. Nous apercevons plus précisément sur les figures 6 et 7 que la première électrode 17 et la deuxième électrode 18 sont agencées au-dessus de la surface supérieure 5 de l'embase 4. La première électrode 17 et la deuxième électrode 18 sont reliées à un générateur de courant électrique (décrit plus loin dans la description) de sorte à produire un courant électrique qui est destiné à provoquer la pénétration du produit à distribuer dans la peau. La première électrode 17 et la deuxième électrode 18 pour iontophorèse sont séparées entre elles par une zone inter-électrode 19 assurant un espacement constant entre la première et la deuxième électrodes 17, 18. La zone inter-électrode 19 comprend une surface d'application 20 destinée à être en contact avec la peau pour appliquer le produit à distribuer. De manière plus précise, la zone inter-électrode 19, est avantageusement, mais non limitativement, formée par un organe d'assemblage ayant une base 21 présentant une paroi latérale 22 reliant une première surface 23 orientée vers la peau de l'utilisateur, laquelle définit ainsi la surface d'application 20 et une deuxième surface 24 qui en est opposée. L'organe d'assemblage présente ici deux pions 25 qui s'étendent depuis la deuxième surface 24 et qui sont destinés à s'emmancher dans deux trous 26 correspondants de l'embase 4 de manière à permettre l'emmanchement de l'organe d'assemblage dans l'embase 4. Les trous 26 traversent l'embase 4 depuis la surface supérieure 5 vers la surface inférieure 6 de l'embase 4 (cf. figure 4). La paroi latérale 22 présentant une certaine épaisseur, la surface d'application 20 est située à distance de la surface supérieure 5 de l'embase 4. En particulier, la surface supérieure 5 de l'embase 4 est définie dans un plan A alors que les première et deuxième électrodes 17, 18, étant disposées au-dessus de la surface supérieure 5, sont définies dans un plan B parallèle au plan A. Quant à la zone inter-électrode 19, et en particulier, la surface d'application 20, celle-ci est définie dans un plan C qui est parallèle au plan B des première et deuxième électrodes 17, 18. En d'autres termes, les premières et les deuxièmes électrodes 17, 18 sont en retrait de ladite zone inter-électrode 19, si bien que lesdites premières et les deuxièmes électrodes ne sont pas en contact avec la peau de l'utilisateur. Le plan B des première et deuxième électrodes 17, 18 et le plan C de la zone inter-électrode 19 sont situés à une distance prédéterminée l'un de l'autre. La distance prédéterminée est comprise entre 0.3 et 1.3 millimètres (mm). Suivant une caractéristique particulière de l'invention, la zone inter-électrode 19 présente une distance dmin minimale prédéterminée entre la première électrode 17 et la deuxième électrode 18 qui est comprise entre 5 et 20 mm. De préférence, mais non limitativement, cette distance dmin minimale est de 10 mm. La cavité de distribution 92 est ainsi obtenue du fait de la distance séparant les plans B, C des première et deuxième électrodes 17, 18 et de la zone inter-électrode 19. Celle-ci présente donc au moins un fond formé par la surface supérieure 5 de l'embase 4 et des parois latérales formées par la paroi latérale 22 de l'organe d'assemblage (zone inter-électrode 19). Les parois latérales de la cavité 92 peuvent être formées également par une paroi interne d'une collerette 38 d'un capuchon 37 que comprend la tête d'application 3 qui est décrit ci-après. La cavité 92 de la tête d'application 3 permet qu'une fine pellicule de produit à distribuer se forme dans cette cavité 92. En d'autres termes, l'accumulation de produit est évitée sur l'interface supérieure de la tête d'application.

De manière avantageuse, mais non limitativement, le moyen de distribution 11 comprend un ou plusieurs orifices de sortie du produit vers la peau de l'utilisateur, lequel débouche sur l'interface supérieure de la tête d'application 3.

Selon une caractéristique de l'invention, la première électrode 17 et la deuxième électrode 18 présentent chacune une paroi 27 munie de perforations 28.

Suivant un premier mode de réalisation illustré sur la figure 6, la ou les perforations 28 distribuent le produit vers la peau de l'utilisateur. En d'autres termes, les perforations 28 constituent la ou les orifices de sortie du produit vers la peau. Ainsi, le produit circule à travers l'orifice de distribution 91 de l'embase 4 vers les perforations 28 de chaque première et deuxième électrodes 17, 18. Dans ce mode de réalisation, les première et deuxième électrodes sont réalisées dans un matériau électriquement conducteur. Ce matériau électriquement conducteur peut comprendre un matériau métallique ou un polymère ou un matériau composite comprenant ce matériau polymère. Le polymère peut être un polytétrafluoroéthylène (PTFE). Le matériau métallique peut être un acier inoxydable.

Suivant un deuxième mode de réalisation illustré sur la figure 7, au moins une plaque 29 présentant une paroi munie de perforations 31 est disposée dans la tête 3 d'application du dispositif 1 d'application. Plus précisément, au-dessus de chaque première et deuxième électrodes 17, 18 est agencée la plaque 29. Dans ce cas de figure, le produit circule à travers la ou les orifice (s) de distribution 91, la ou les perforations 28 des première et deuxième électrodes 17, 18 vers les perforations 31 de la plaque 29 lesquelles distribuent le produit vers la peau de l'utilisateur. De manière avantageuse, cette plaque 29 est non conductrice de sorte à éviter les irritations ou picotements sur la peau de l'utilisateur. La plaque 29 peut être réalisée dans un matériau polymère. De préférence, mais non limitativement, le polymère est un thermoplastique tel qu'un polytétrafluoroéthylène (PTFE) ou un polyoxyméthylène (POM). Dans ce mode de réalisation, les première et deuxième électrodes 17, 18 pour iontophorèse sont réalisées dans un matériau composite. De manière avantageuse, le matériau composite peut être un polymère chargé en carbone.

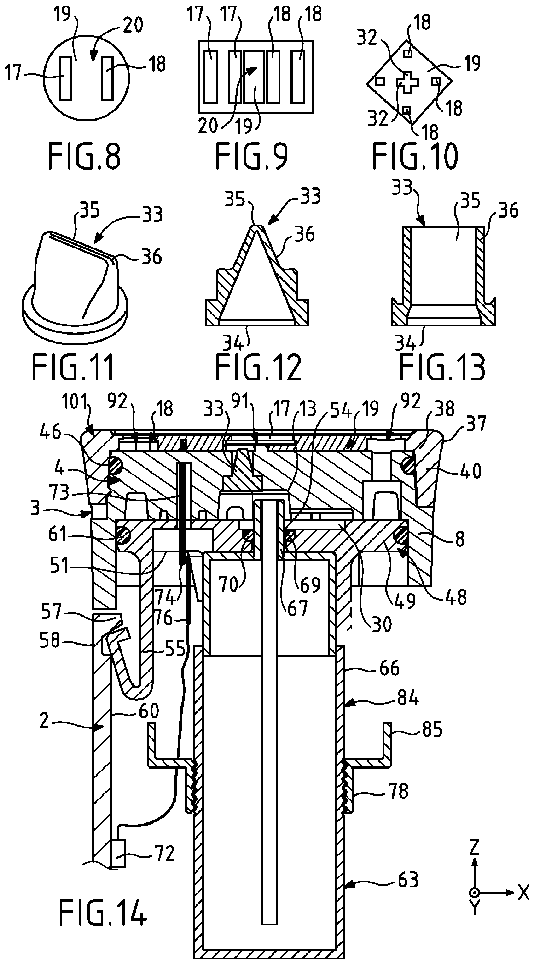

Les premières et deuxièmes électrodes 17, 18 peuvent être agencées de diverses manières au-dessus de la surface supérieure 5 de l'embase 4. Dans un mode de réalisation, tel qu'illustré sur les figures 1, 3, 6, et 7, la première électrode 17 est disposée au centre de l'embase 4. Quant à la deuxième électrode 18, celle-ci est disposée à la périphérie de la tête d'application 3, et de manière plus précise, autour du périmètre de la zone inter-électrode 19. La première électrode 17 illustrée, présente une forme sensiblement triangulaire. La deuxième électrode 18 présente, dans ce mode de réalisation, un contour fermé avec une forme générale triangulaire. Dans un autre mode de réalisation, illustré sur la figure 8, les première et deuxième électrodes 17, 18 pour iontophorèse présentent une forme rectangulaire. Celles-ci sont disposées parallèlement et sont séparées par la zone inter-électrode 19. Selon encore un autre mode réalisation illustré sur la figure 9, les première et deuxième électrodes 17, 18 sont de forme rectangulaire. Celles-ci sont au nombre de quatre et sont disposées à distance l'une de l'autre. La zone inter-électrode 19 est disposée au centre de la tête d'application 3 de sorte à avoir, par exemple, deux premières électrodes 17 à gauche de la zone inter-électrode et deux deuxièmes électrodes 18 à droite de la zone inter-électrode 19. Selon encore un autre moyen de réalisation illustré sur la figure 10, la première électrode 17 présente une forme générale en croix présentant deux branches perpendiculaires formant alors quatre bords 32. La première électrode 17 est disposée au centre de la tête 3, et en particulier au centre de la surface supérieure 5 de l'embase 4. Des deuxièmes électrodes 18, ici quatre, sont disposées à distance de la première électrode 17 et autour de celle-ci. Chaque deuxième électrode 18 est disposée dans le prolongement d'un bord 32 de la première électrode 17. La zone inter-électrode 19 sépare la première et les deuxièmes électrodes 17, 18. D'autres configurations des première et deuxièmes électrodes 17, 18 sont, bien entendu, envisageables.

Dans les divers modes de réalisation décrits, la tête 3 d'application comprend au moins un moyen d'isolation 33 électrique qui est interposé fluidiquement entre la première électrode 17 et la deuxième électrode 18. Ce moyen d'isolation 33 électrique est configuré de manière à autoriser ou limiter, voire interdire le passage du courant électrique entre la première électrode 17 et la deuxième électrode 18 via la tête d'application 3. En référence aux figures 6 et 7, le moyen d'isolation 33 est disposé sur un des canaux de distribution 16. Afin de pouvoir contrôler la circulation du courant électrique entre la première électrode 17 et la deuxième électrode 18, le moyen d'isolation 33 électrique est disposé sur le canal de distribution 16 qui mène vers la première électrode 17, entre l'orifice de distribution 91 et la sortie 15 du réservoir tampon 13. En effet, sur les figures 6 et 7, la première électrode 17 pour iontophorèse est située au centre de la tête d'application 3 alors que la deuxième électrode 18 pour iontophorèse entoure la périphérie de la première électrode 17, et en particulier, la zone inter-électrode 19.

De manière avantageuse, mais non limitativement, le moyen d'isolation 33 électrique est situé à proximité de l'orifice de distribution 91 du canal de distribution 16 menant vers la première électrode 17 afin de limiter au maximum les courants de fuite vers la deuxième électrode 18 via la tête d'application.

Pour autoriser ou limiter le passage du courant entre la première électrode 17 et la deuxième électrode 18 via la tête d'application 3, le moyen d'isolation 33 électrique est apte à occuper une position ouverte et une position fermée. Dans la position fermée le courant circule uniquement entre la première électrode 17 et la deuxième électrode 18 et dans la position ouverte le courant circule de manière limitée dans la tête d'application 3 et dans le corps 2. Le moyen d'isolation 33 électrique présente une section de passage, qui, lorsque le moyen d'isolation 33 électrique occupe la position ouverte, permet la circulation du courant électrique entre la première et la deuxième électrodes 17, 18 et dans les canaux de distribution 16 de la tête d'application 3. Néanmoins, cette section de passage est tellement petite que l'impédance du chemin à parcourir est trop importante pour que la circulation du courant soit privilégiée via cette section de passage. Ainsi, le courant électrique circule quasi exclusivement dans l'interface d'application (coté peau) plutôt que vers l'intérieur de la tête d'application 3 et le corps 2.

Dans la position ouverte, le produit à distribuer circule depuis le corps 2 vers les première et deuxième électrodes 17, 18 alors que dans la position fermée le produit ne circule plus vers la première électrode 17. Dès que du produit est de nouveau extrait depuis le moyen de stockage 84 vers le réservoir tampon 13, la pression du produit provoque l'ouverture du moyen d'isolation 33 électrique de manière à ce que les première et deuxièmes électrodes 17, 18 pour iontophorèse soient alimentées en produit. La pression d'ouverture du moyen d'isolation 33 électrique est choisie de manière adaptée par rapport aux canaux de distribution 16 alimentant la deuxième électrode 18 et la perte de charge associée. Idéalement, la perte de charge subie par le produit à distribuer au passage du moyen d'isolation 33 est égale à la perte de charge des canaux de distribution 16 plus longs alimentant la deuxième électrode 18. De manière alternative, des moyens d'isolation 33 électrique peuvent être également disposés sur les canaux de distribution 16 menant vers la deuxième électrode 18 pour iontophorèse. Cela permet avantageusement d'équilibrer la circulation du produit vers les première et deuxième électrodes 17, 18 pour iontophorèse, et ainsi de pouvoir optimiser l'égalité d'alimentation en produit des première et deuxième électrodes 17, 18 pour iontophorèse.

Par ailleurs, le moyen d'isolation 33 prend la position ouverte lorsqu'une pression à l'intérieur du moyen d'isolation 33 atteint un seuil prédéterminé. De manière avantageuse, mais non limitativement, le moyen d'isolation 33 est un clapet, et de préférence, un clapet de non retour tel qu'illustré sur les figures 11 à 13. Cependant, le moyen d'isolation 33 peut prendre une autre forme. Le clapet comprend un corps présentant une première extrémité au niveau de laquelle est formée une entrée 34 et une deuxième extrémité opposée au niveau de laquelle est formée une sortie 35. Cette dernière 35 se présente sous la forme d'un bec de canard formé de deux lèvres 36 opposées et élastiquement déformables. Les deux lèvres 36 s'ouvrent si la valeur de pression à l'intérieur du clapet est au moins égale à une valeur seuil de pression prédéterminée et se ferment quand la valeur de pression à l'intérieur du clapet est inférieure à la valeur seuil de pression prédéterminée. La valeur seuil de pression prédéterminée est comprise entre 5 et 200 millibars (mbar). De préférence, mais non limitativement, la valeur seuil de pression prédéterminée est de l'ordre de 50 mbar. Il est à noter que dans la position ouverte, la section de passage des lèvres 36 formée au niveau de la sortie 35 est très faible si bien que l'impédance est élevée. La section de passage maximale (d'ouverture) des lèvres 36 est comprise entre 0,2 mm² et 2,5 mm². Ainsi, la circulation du produit à distribuer est possible mais celle du courant est limitée entre la première électrode 17 et la deuxième électrode 18 pour iontophorèse via la tête d'application 3.

Le clapet est réalisé dans un matériau polymère, par exemple un silicone.

La tête 3 d'application comporte le capuchon 37 ou couvercle solidarisé à l'embase 4 pour maintenir les première et deuxième électrodes 17, 18 en position dans la tête d'application 3. Le capuchon 37 comprend la collerette 38 qui présente une ouverture centrale 39 par laquelle l'embase 4 est reçue. Les première et deuxième électrodes 17, 18 ainsi que l'organe d'assemblage (surface d'application 20) sont visibles à travers cette ouverture centrale 39. La collerette 38 présente une surface supérieure 101 qui est définie dans un plan D. Cette surface 101 supérieure forme un anneau d'appui qui a pour but de créer un volume avec une faible épaisseur sur la peau. Ce plan D est sensiblement parallèle au plan C de la surface d'application 20 de la zone inter-électrode 19. Le plan D est également parallèle au plan B des première et deuxième électrodes 17, 18 pour iontophorèse. La collerette 38 du capuchon 37 comprend à sa périphérie une jupe latérale 40 destinée à coopérer avec la paroi 7 de l'embase 4. En particulier, entre la jupe latérale 8 et la paroi 7 de l'embase 4 est prévu un épaulement 41 sur lequel prend appui une extrémité libre 42 de la jupe latérale 40. La paroi 7 de l'embase 4 présente également des encoches 43 aveugles (cf. figure 5) dans chacune desquelles est clippée une saillie 44 prévue sur une paroi interne 45 de la jupe latérale 40 pour fixer le capuchon 37 sur l'embase 4. Pour faciliter le démontage du couvercle 37 de l'embase 4, celle-ci comprend sur la paroi 7 une entaille 99. Cette dernière est située à proximité de chaque encoche 43 aveugle et permet qu'un doigt d'un utilisateur accède à l'extrémité libre 42 du couvercle 37.

Entre la paroi interne 45 de la jupe latérale 40 du capuchon 37 et la paroi 7 de l'embase 4 est agencé un premier joint d'étanchéité 46. Afin que le premier joint d'étanchéité 46 soit maintenu en position, la paroi 7 de l'embase 4 comprend une gorge 47 s'étendant dans le sens du périmètre de la paroi 7. Le premier joint d'étanchéité 46 possède un corps annulaire. Avantageusement, le premier joint d'étanchéité 46 est réalisé dans un matériau déformable élastiquement. Ce matériau déformable est de préférence un polymère ou un copolymère choisi parmi un éthylène-propylène-diène monomère (EPDM), un caoutchouc fluorocarboné (FPM), un élastomère polyacrylique (ACM), un copolymère éthylène acrylique (AEM), un caoutchouc nitrile hydrogéné (HNBR), un VITON ®, ou encore un butyle.

La tête d'application 3 comporte en outre un organe de connexion 48 pour connecter la tête 3 d'application et le corps 2 de manière amovible (cf. figure5). Cet organe de connexion 48 comprend une partie de support 49 laquelle comprend une face supérieure 50 et une face inférieure 51 reliées par une paroi 52. La partie de support 49 présente une lumière 54 traversant la paroi 52 de part et d'autre. Cette lumière 54 communique avec l'entrée 14 du réservoir tampon 13. La partie de support 49 est équipée de pattes de fixation 55, ici il y en a trois, qui s'étendent depuis la face inférieure 51 de la partie de support 49. La partie de support 49 s'emboite à force avec la surface interne 9 de la jupe latérale 8 de l'embase 4. Les pattes de fixation 55 présentent vers chacune de leur extrémité libre 56 une forme sensiblement en V. Ces pattes de fixation 55 coopèrent avec un renflement 57 prévu à une extrémité proximale 58 du corps 2. Le renflement 57 est agencé sur une surface interne 60 du corps 2. La paroi 52 est pourvue d'une gorge 53 s'étendant le long du périmètre de la paroi 52 et dans laquelle est installé un second joint d'étanchéité 61. Ce dernier 61 est en contact avec la surface interne 9 de la jupe latérale 8.

De manière avantageuse, le produit à distribuer est contenu dans une cartouche 63 qui est connectée de manière amovible au corps 2 du dispositif. Ainsi, lorsque le produit est épuisé, la cartouche 63 peut être remplacée facilement ou être à nouveau remplie.

En référence à la figure 14, la cartouche 63 comprend un boitier 66 recevant une tête de fixation 67. La tête de fixation 67 est introduite à travers la lumière 54 de l'organe de connexion 48 et une portion de celle-ci s'étend dans le réservoir tampon 13.La tête de fixation 67 est fixe par rapport à la tête d'application 3. Un joint d'étanchéité 70 est également logé dans une gorge 69 de la partie de support 49 de l'organe de connexion 48. Le joint d'étanchéité 70 est plaqué dans la gorge 69 par une butée 30 de sorte que le produit à distribuer ne coupe pas la tête de fixation 67 de la cartouche 63.

Le corps 2 comprend au moins un moyen d'extraction 78 permettant d'extraire le produit du moyen de stockage 84 depuis le corps 2. Ce moyen 78 peut être manuel ou motorisé. En mode manuel, l'appareil peut comprendre un moyen d'entrainement comprenant une portion 83 (cf. figure 1) s'étendant à travers l'ouverture 77 du corps 2 du dispositif 1 de sorte que l'utilisateur l'actionne facilement. De manière avantageuse, mais non limitativement, ce moyen d'entrainement 82 comprend une molette 85.

En variante, le moyen d'extraction 78 motorisé comprend un moteur (non représenté) actionnant des moyens permettant d'entrainer en rotation un mécanisme de distribution . Le moteur peut être actionné via un bouton accessible sur le corps 2 du dispositif 1 d'application. Ce bouton est relié à un circuit électronique de commande 72 pour actionner le moteur.

Dans le corps 2 est également agencé un générateur de courant 71 permettant de délivrer un courant de faible intensité aux première et deuxième électrodes 17, 18 disposées dans la tête 3 d'application. Le courant électrique est compris entre 50 microampères (µA) et 6000 µA. De préférence, mais non limitativement le courant électrique est de l'ordre de 800 µA. Le courant peut être alternatif ou continu. Le générateur de courant 71 électrique est commandé par le circuit électronique de commande 72 installé dans le corps 2 du dispositif 1 d'application de produit pour fournir le courant. Le circuit électronique de commande 72 est alimenté par une source d'alimentation 90 électrique pouvant être une pile ou une batterie rechargeable sur un réseau électrique domestique. De manière alternative, le dispositif 1 peut comporter un cordon d'alimentation électrique (non représenté) destiné à être branché sur le réseau électrique domestique et à alimenter le circuit électronique de commande 72. Afin d'assurer une connexion électrique entre le générateur de courant 71 et les première et deuxième électrodes 17, 18, ces dernières sont dotées chacune d'une broche 73, 73'. Les broches 73, 73' et les première et deuxième électrodes 17, 18 sont formées d'une seule pièce. Chaque broche 73, 73' (cf. figures 5 et 14) présente une extrémité libre 74 accessible depuis l'interface inférieure de la tête d'application 3. En d'autres termes, chaque broche 73, 73' traverse la zone inter-électrode 19, l'embase 4 et l'organe de connexion 48 de part et d'autre via des fentes 75, 75' que comprennent ceux-ci. Les broches 73, 73' sont donc accessibles depuis la face inférieure 51 de l'organe de connexion 48 de la tête d'application 3. Chaque broche 73, 73' présente une longueur supérieure à celles de la zone inter-électrode, de l'embase 4 et de l'organe de connexion 48. Ici, chacune des broches 73, 73' présente une section en forme de L inversé. Le corps 2 du dispositif comprend des éléments de connexion 76 qui sont reliés au générateur de courant 71.

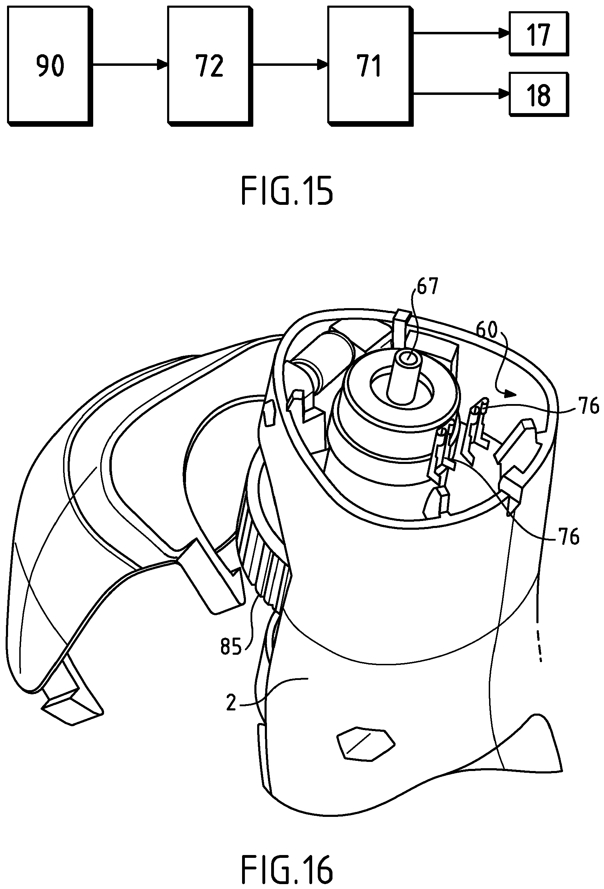

Sur les figures 14 à 16, un élément de connexion 76 est connecté à l'anode du générateur de courant et un autre élément de connexion 76 est connecté à la cathode du générateur de courant 71. De la sorte, lorsque la tête d'application 3 est connectée avec le corps 2 du dispositif 1, chaque broche 73, 73' coopère avec un élément de connexion 76 et est alimentée en courant électrique.

Selon une caractéristique de l'invention, lorsque du produit est distribué, un circuit fermé est formé avec la peau de l'utilisateur, la première électrode 17 et la deuxième électrode 18. Afin d'éviter des microlésions de la peau créant alors des sensations d'inconfort, voire de douleurs en cas de forte intensité ou de durée prolongée, ou pour détecter une anomalie de fonctionnement ou de tolérance, le dispositif peut comprendre une sonde de température. De manière avantageuse, cette sonde de température est intégrée dans la tête d'application 3 du dispositif d'application 1 et reliée au circuit électronique de commande 72.

Nous allons maintenant décrire le fonctionnement du dispositif d'application. Lorsque le dispositif d'application est mis sous tension, le circuit électronique de commande 72 adresse un ordre au générateur de courant 71 lequel génère un courant aux première et deuxième électrodes 17, 18 pour iontophorèse. L'utilisateur actionne le moyen d'extraction 78 jusqu'à ce que le produit soit extrait de la cartouche 63 et collecté dans un premier temps dans le réservoir tampon 13. Dans un deuxième temps, le produit sous pression déclenche l'ouverture du clapet, lorsque la pression à l'intérieur de celui atteint une valeur prédéterminée, ce qui permet le passage du produit dans les canaux de distribution 16 formés dans l'embase 4. Le produit débouche alors dans la cavité de distribution 92 via les orifices de distribution 91 vers les première et deuxièmes électrodes 17, 18. Le produit traversant les perforations 28 des première et deuxième électrodes 17, 18 au droit du champ électrique créé est transporté en profondeur de la peau suivant les lignes de champs en arc en demi-cercle. La distance atteinte sous la peau est comprise entre 2 et 10 mm de profondeur.

L'invention est décrite dans ce qui précède à titre d'exemple. Il est entendu que la personne de l'art est à même de réaliser différentes variantes de réalisation de l'invention, en associant par exemple les différentes caractéristiques ci-dessus prises seules ou en combinaison, sans pour autant sortir du cadre de l'invention.

## Revendications

1. Dispositif (1) d'application d'un produit à distribuer sur une peau d'un utilisateur par iontophorèse comprenant:
- un corps (2) destiné à recevoir le produit à distribuer comprenant un générateur de courant (71) électrique; et,
- une tête d'application (3) montée sur le corps (2), la tête d'application (3) comportant :
∘ au moins un moyen de distribution (11) du produit à distribuer sur la peau ; et
∘ au moins une première (17) et une deuxième électrodes (18) séparées par une zone inter-électrode (19), **caractérisées en ce que** les premières et les deuxièmes électrodes (17, 18) sont en retrait de ladite zone inter-électrode et reçoivent un courant électrique dudit générateur (71).

2. Dispositif selon la revendication 1, dans lequel les première et deuxième électrodes (17, 18) sont situées dans un même plan (B).

3. Dispositif (1) selon la revendication 2, dans lequel la zone inter-électrode (19) est située dans un plan (C) parallèle audit plan (B) des première et deuxième électrodes (17, 18), le plan (B) des première et deuxième électrodes (17, 18) et le plan de la zone inter-électrode étant situés à une distance prédéterminée l'un par rapport l'autre.

4. Dispositif (1) selon l'une quelconque des revendications précédentes, dans lequel la zone inter-électrode (19) comprend une surface d'application (20) destinée à être en contact avec la peau de l'utilisateur.

5. Dispositif (1) selon l'une quelconque des revendications précédentes, dans lequel la zone inter-électrode (19) présente une distance minimale (dmin) prédéterminée entre la première électrode (17) et la deuxième électrode (18) qui est comprise entre 5 et 20 mm.

6. Dispositif (1) selon l'une quelconque des revendications précédentes, dans lequel la tête d'application (3) comprend un circuit de distribution (12) comprenant des canaux de distribution (16) distribuant le produit vers le moyen de distribution (11) du produit.

7. Dispositif (1) selon la revendication 6, dans lequel la première électrode (17) et la deuxième électrode (18) sont agencées en aval du circuit de distribution (12).

8. Dispositif (1) selon l'une quelconque des revendications précédentes, dans lequel la première électrode (17) et la deuxième électrode (18) comprennent chacune une paroi munie de perforations (28) et en ce que le moyen de distribution (11) comprend un ou des orifices de sortie, les perforations (28) constituant le ou les orifices de sortie du produit vers la peau de l'utilisateur.

9. Dispositif (1) selon l'une quelconque des revendications 1 à 7, dans lequel une plaque (29) munie de perforations (31) est agencée dans la tête d'application (3), le moyen de distribution (11) comprenant un ou des orifices de sortie et les perforations (31) constituant le ou les orifices de sortie du produit vers la peau de l'utilisateur.

10. Dispositif (1) selon l'une quelconque des revendications précédentes, dans lequel le produit est contenu dans une cartouche (63) connectée de manière amovible au corps (2) du dispositif (1).

11. Dispositif (1) selon l'une quelconque des revendications précédentes, dans lequel le corps (2) comprend au moins un moyen d'extraction (78) du produit du corps (2) vers la tête d'application (3).

12. Dispositif (1) selon l'une quelconque des revendications précédentes, dans lequel la tête d'application (3) comprend un moyen d'isolation(33) électrique lequel est interposé fluidiquement entre la première électrode (17) et la deuxième électrodes (18), ledit moyen d'isolation (33) électrique étant configuré de manière à autoriser ou interdire le passage du courant électrique depuis les première et deuxième électrodes (17, 18) vers le corps.

13. Dispositif (1) selon la revendication 12, dans lequel le moyen d'isolation (33) électrique est propre à occuper:
- une position fermée dans laquelle le courant circule uniquement entre les première et deuxième électrodes (17, 18); et,
- une position ouverte dans laquelle le courant circule entre les première et deuxième électrodes (17, 18) et dans les canaux de distribution (16),
et en ce que le moyen d'isolation (33) électrique prend la position ouverte lorsqu'une pression à l'intérieur du moyen d'isolation (33) électrique atteint un seuil prédéterminé.

14. Dispositif (1) selon l'une quelconque des revendications 12 ou 13, dans lequel le moyen d'isolation (33) électrique est disposé sur un des canaux de distribution menant vers la première électrode (17) .

15. Dispositif (1) selon l'une quelconque des revendications précédentes, dans lequel la première électrode (17) est disposée au centre de la tête (3) et la deuxième électrode (18) est disposée vers la périphérie de la tête (3).

## Patentansprüche

1. Vorrichtung (1) zum Auftragen eines Produkts auf einer Haut eines Benutzers durch lonophorese, die Folgendes umfasst:
- einen Körper (2), der dazu bestimmt ist, das zu verteilenden Produkt aufzunehmen, der einen Generator (71) elektrischen Stroms umfasst; und
- einen Auftragkopf (3), der auf den Körper (2) montiert ist, wobei der Auftragkopf (3) Folgendes umfasst:
∘ mindestens ein Verteilungsmittel (11) des auf der Haut zu verteilenden Produkts; und
∘ mindestens eine erste (17) und eine zweite Elektrode (18), die durch eine Zwischenelektrodenzone (19) getrennt sind, **dadurch gekennzeichnet, dass** die erste und die zweite Elektrode (17, 18) von der Zwischenelektrodenzone nach hinten versetzt sind und einen Strom von dem Generator (71) empfangen.

2. Vorrichtung nach Anspruch 1, wobei die erste und die zweite Elektrode (17, 18) in einer selben Ebene (B) liegen.

3. Vorrichtung (1) nach Anspruch 2, wobei die Zwischenelektrodenzone (19) in einer Ebene (C) parallel zu der Ebene (B) der ersten und zweiten Elektrode (17, 18) liegt, wobei die Ebene (B) der ersten und zweiten Elektrode (17, 18) und die Ebene der Zwischenelektrodenzone in einem vorbestimmten Abstand voneinander liegen.

4. Vorrichtung (1) nach einem der vorstehenden Ansprüche, wobei die Zwischenelektrodenzone (19) eine Auftragoberfläche (20) umfasst, die dazu bestimmt ist, mit der Haut des Benutzers in Berührung zu sein.

5. Vorrichtung (1) nach einem der vorstehenden Ansprüche, wobei die Zwischenelektrodenzone (19) einen vorbestimmten Mindestabstand (dmin) zwischen der ersten Elektrode (17) und der zweiten Elektrode (18) aufweist, der zwischen 5 und 20 mm liegt.

6. Vorrichtung (1) nach einem der vorstehenden Ansprüche, wobei der Auftragkopf (3) einen Verteilungskreislauf (12) umfasst, der Verteilungskanäle (16) umfasst, die das Produkt zu dem Verteilungsmittel (11) des Produkts verteilen.

7. Vorrichtung (1) nach Anspruch 6, wobei die erste Elektrode (17) und die zweite Elektrode (18) stromabwärts des Verteilungskreislaufs (12) eingerichtet sind.

8. Vorrichtung (1) nach einem der vorstehenden Ansprüche, wobei die erste Elektrode (17) und die zweite Elektrode (18) jeweils eine Wand umfassen, die mit Perforierungen (28) versehen ist, und dass das Verteilungsmittel (11) eine oder mehrere Ausgangsöffnungen umfasst, wobei die Perforierungen (28) die Ausgangsöffnung(en) des Produkts zu der Haut des Benutzers bilden.

9. Vorrichtung (1) nach einem der Ansprüche 1 bis 7, wobei eine Platte (29), die mit Perforierungen (31) versehen ist, in dem Auftragkopf (3) eingerichtet ist, wobei das Verteilungsmittel (11) eine Öffnung(en) (31) umfasst, die die Ausgangsöffnung(en) des Produkts zu der Haut des Benutzers bilden.

10. Vorrichtung (1) nach einem der vorstehenden Ansprüche, wobei das Produkt in einer Patrone (63) enthalten ist, die abnehmbar mit dem Körper (2) der Vorrichtung (1) verbunden ist.

11. Vorrichtung (1) nach einem der vorstehenden Ansprüche, wobei der Körper (2) mindestens ein Mittel (78) zum Extrahieren des Produkts aus dem Körper (2) zu dem Auftragkopf (3) umfasst.

12. Vorrichtung (1) nach einem der vorstehenden Ansprüche, wobei der Auftragkopf (3) ein Mittel (33) zum elektrischen Isolieren umfasst, das fluidisch zwischen der ersten Elektrode (17) und der zweiten Elektrode (18) eingefügt ist, wobei das Mittel (33) zum elektrischen Isolieren derart konfiguriert ist, dass es das Durchgehen des elektrischen Stroms von der ersten und zweiten Elektrode (17, 18) zu dem Körper gestattet oder untersagt.

13. Vorrichtung (1) nach Anspruch 12, wobei das Mittel (33) zum elektrischen Isolieren geeignet ist, um:
- eine geschlossene Position einzunehmen, in der der Strom nur zwischen der ersten und zweiten Elektrode (17, 18) zirkuliert; und
- eine offene Position einzunehmen, in der der Strom zwischen der ersten und zweiten Elektrode (17, 18) und in den Verteilungskanälen (16) zirkuliert,
und dass das Mittel (33) zum elektrischen Isolieren die offene Position einnimmt, wenn ein Druck in dem Inneren des Mittels (33) zum elektrischen Isolieren einen vorbestimmten Schwellenwert erreicht.

14. Vorrichtung (1) nach einem der Ansprüche 12 oder 13, wobei das Mittel (33) zum elektrischen Isolieren auf einem der Verteilungskanäle, die zu der ersten Elektrode (17) führen, angeordnet ist.

15. Vorrichtung (1) nach einem der vorstehenden Ansprüche, wobei die erste Elektrode (17) in der Mitte des Kopfs (3) angeordnet ist, und die zweite Elektrode (18) zum Umfang des Kopfs (3) hin angeordnet ist.

## Claims

1. Device (1) for applying a product to be distributed on the skin of a user by iontophoresis comprising:
- a body (2) intended to receive the product to be distributed comprising an electric current generator (71); and,
- an applicator head (3) mounted on the body (2), the applicator head (3) comprising:
∘ at least one distribution means (11) for distributing the product to be distributed on the skin; and
∘ at least one first electrode (17) and one second electrode (18) separated by an inter-electrode zone (19), **characterised in that** the first and second electrodes (17, 18) being set back from said inter-electrode zone and receive an electric current from said generator (71).

2. Device according to claim 1, wherein the first and second electrodes (17, 18) are situated in a same plane (B).

3. Device (1) according to claim 2, wherein the inter-electrode zone (19) is situated in a plane (C) parallel to said plane (B) of the first and second electrodes (17, 18), the plane (B) of the first and second electrodes (17, 18), and the plane of the inter-electrode zone being situated at a predetermined distance from each other.

4. Device (1) according to any one of the preceding claims, wherein the inter-electrode zone (19) comprises an application surface (20) intended to be in contact with the user's skin.

5. Device (1) according to any one of the preceding claims, wherein the inter-electrode zone (19) has a predetermined minimum distance (dmin) between the first electrode (17) and the second electrode (18) which is between 5 and 20mm.

6. Device (1) according to any one of the preceding claims, wherein the applicator head (3) comprises a distribution circuit (12) containing distribution channels (16) for distributing the product toward the distribution means (11) of the product.

7. Device (1) according to claim 6, wherein the first electrode (17) and the second electrode (18) are arranged downstream from the distribution circuit (12).

8. Device (1) according to any one of the preceding claims, wherein the first electrode (17) and the second electrode (18) each comprise a wall with perforations (28) and in that the distribution means (11) comprises the one or more outlet openings, the perforations (28) constituting the product outlet openings toward the user's skin.

9. Device (1) according to any one of claims 1 to 7, wherein a plate (29) with perforations (31) is arranged in the applicator head (3), the distribution means (11) comprising one or more outlet openings and the perforations (31) constituting the one or more product outlet openings toward the user's skin.

10. Device (1) according to any one of the preceding claims, wherein the product is contained in a cartridge (63) removably connected to the body (2) of the device (1).

11. Device (1) according to any one of the preceding claims, wherein the body (2) comprises at least one means for extracting (78) the product from the body (2) toward the applicator head (3).

12. Device (1) according to any one of the preceding claims, wherein the applicator head (3) comprises an electrical insulation means (33) which is fluidically interposed between the first electrode (17) and the second electrode (18), said electrical insulation means (33) being configured so as to permit or prevent the passage of electric current from the first and second electrodes (17, 18) to the body.

13. Device (1) according to claim 12, wherein the electrical insulation means (33) is adapted to be in:
- a closed position in which current circulates only between the first and second electrodes (17, 18); and
- an open position in which current circulates between the first and second electrodes (17, 18) and in distribution channels (16), and in that the electrical insulation means (33) is in the open position when a pressure inside the electrical insulation means (33) reaches a predetermined threshold.

14. Device (1) according to any one of claims 12 or 13, wherein the electrical insulation means (33) is arranged on one of the distribution channels leading to the first electrode (17).

15. Device (1) according to any one of the preceding claims, wherein the first electrode (17) is arranged at the centre of the head (3) and the second electrode (18) is arranged toward the periphery of the head (3).
